# EUROPÄISCHE PATENTSCHRIFT

(11) **EP 2 759 270 B1**
(45) Veröffentlichungstag und Bekanntmachung des Hinweises auf die Patenterteilung: **29.08.2018**
(21) Anmeldenummer: 14152440.5
(22) Anmeldetag: 24.01.2014
(51) Int. Cl.: A61B 17/29, A61B 17/00

(54) **Medizinisches Instrument**
Medical instrument
Instrument médical

(30) Priorität: 25.01.2013 DE 102013100759
(43) Veröffentlichungstag der Anmeldung: 30.07.2014
(73) Patentinhaber: Karl Storz SE & Co. KG, 78532 Tuttlingen (DE)
(72) Erfinder: Kärcher, Daniel, 78532 Tuttlingen (DE); Merz, Robin, 78532 Tuttlingen (DE)

(56) Entgegenhaltungen:
- EP-A1- 2 316 358
- EP-A1- 2 489 315
- DE-A1-102010 024 136
- US-A- 5 176 702
- US-A1- 2011 166 575

## Beschreibung

Die Erfindung betrifft ein medizinisches Instrument mit einem Schaft, an dessen proximalem Ende eine Handhabe angeordnet ist, wobei der Schaft um seine Längsachse gegenüber der Handhabe verdrehbar gelagert ist, beziehungsweise ein distales Schafthauptteil gegenüber einem proximalen Teil des Schaftes, der als ein Verbindungselement wirkt, verdrehbar ist, und über einen Arretiermechanismus in verschiedenen Drehstellungen relativ zur Handhabe festlegbar ist.

Medizinische Instrumente mit einem um seine Längsachse drehbaren Schaft sind insbesondere bei Instrumenten mit einem zumindest teilweise gebogenen Schaft aus der Praxis bekannt.

Bei diesen bekannten Instrumenten erfolgt die Fixierung des Schaftes mittels Reibschluss oder Formschluss. Die Formschluss-Fixierungen, bei denen miteinander korrespondierende Formteile ineinandergreifen, weisen in der Regel nur eine geringe Anzahl an Verstellstufen auf.
Das Dokument EP 2 489 315 A1 offenbart ein medizinisches Instrument mit einem drehbarem Schaft und einem Rastmechanismus mit einer Lochscheibe und Rastelementen, wobei die Rastelemente in Ausnehmungen in der Lochscheibe eingreifen und in Richtung auf die Lochscheibe federbelastet sind.

Die Reibschluss-Fixierung des Schaftes weist zwar den Vorteil auf, dass das Verdrehen des Schaftes stufenlos erfolgen kann, jedoch weist diese Art der Fixierung auch die Nachteile auf, dass einerseits ein relativ hoher Kraftaufwand erforderlich ist, um die Bauteile fixierend gegeneinander zu verspannen und andererseits die Reibschluss-Fixierung sehr verschleißanfällig ist und darüber hinaus Funktionsprobleme bei Feuchtigkeit aufweisen kann.

Davon ausgehend liegt der Erfindung die **Aufgabe** zugrunde, ein medizinisches Instrument mit einem um seine Längsachse drehbaren Schaft so auszugestalten, dass dieses Instrument bei einfacher Handhabung eine Vielzahl sicherer und positionsgenauer Drehungen des Schaftes gegenüber der Handhabe ermöglicht, gleichzeitig jedoch einen möglichst geringen Bauraum einnimmt.

Die **Lösung** dieser Aufgabenstellung wird erfindungsgemäß durch die Merkmale des unabhängigen Anspruchs 1 gelöst, mit bevorzugten Ausführungsformen entsprechend den abhängigen Ansprüchen. Der Arretiermechanismus ist als Rastmechanismus ausgebildet ist, der mindestens eine mit Ausnehmungen versehene Lochscheibe sowie Rastelemente aufweist, wobei die Rastelemente und/oder die mindestens eine Lochscheibe relativ zueinander federbelastet sind und mindestens ein Rastelement zum Fixieren des Schaftes in eine entsprechende Ausnehmung der mindestens einen Lochscheibe eingreift. In einer nicht erfindungsgemäßen Alternative zu der Lochscheibe könnte auch eine Platte mit einer Kontur, z.B. strahlenförmig beziehungsweise radial verlaufenden Ausnehmungen, verwendet werden. Dabei würde wiederum mindestens ein Rastelement zum Fixieren des Schaftes in eine entsprechende Ausnehmung bzw. einen Rücksprung in der Kontur der Lochscheibe eingreifen.

Die Ausbildung des Arretiermechanismus als Rastmechanismus mit federbelasteten Rastelementen, die in Ausnehmungen einer Lochscheibe eingreifen, stellt eine einfach und sicher zu handhabende Verstell- und Fixiermöglichkeit zum Verdrehen des Schaftes dar, die eine Vielzahl von positionsgenauen Fixierungsstufen ermöglicht.

Um die Anzahl an Raststufen bei gleicher Lochscheibe zu erhöhen, wird gemäß der Erfindung vorgeschlagen, dass die Rastelemente in Richtung auf die mindestens eine Lochscheibe federbelastet sind und, dass der Abstand zweier nebeneinander angeordneter Rastelemente voneinander sich vom Abstand zweier nebeneinander angeordneter Ausnehmungen der mindestens einen Lochscheibe unterscheidet. Bei der Ausbildung des Abstandes zweier nebeneinanderliegender Rastelemente voneinander auf den halben Abstand zweier nebeneinander angeordneter Ausnehmungen der Lochscheibe verdoppelt sich die Anzahl der mit dieser Lochscheibe zu verwirklichenden Fixierungsstufen. In dieser Ausführungsform rasten immer nur die Hälfte der Rastelemente in die Ausnehmungen ein.

Gemäß einer praktischen Ausführungsform der Erfindung wird vorgeschlagen, dass in der den Schaft fixierenden Stellung des Arretiermechanismus immer ein aus zwei Rastelementen bestehendes Rastelementepaar in zwei entsprechende Ausnehmungen der mindestens einen Lochscheibe eingreift, um eine stabile und torsionssteife Fixierung zu gewährleisten. Vorteilhafterweise weist der Arretiermechanismus mehrere nebeneinander angeordnete Rastelementepaare auf, um mehrere Fixierungsstufen zu ermöglichen.

Auch bei der Verrastung mittels der paarweise in die Ausnehmungen der Lochscheibe eingreifenden Rastelementepaare wird mit der Erfindung vorgeschlagen, dass der Abstand zweier nebeneinander angeordneter Rastelementepaare voneinander sich vom Abstand zweier nebeneinander angeordneter Ausnehmungen der mindestens einen Lochscheibe unterscheidet, damit in Abhängigkeit der ungleichen Abstände einerseits der Rastelementepaare voneinander und andererseits der Ausnehmungen der Lochscheibe voneinander lässt sich die Anzahl der möglichen Fixierungsstufen bei gleicher Lochscheibe deutlich erhöhen.

Um den Durchmesser der medizinischen Instrumente auch im Bereich des Arretiermechanismus möglichst gering ausbilden zu können, wird mit der Erfindung vorgeschlagen, dass die Ausnehmungen der mindestens einen Lochscheibe in Axialrichtung des Schaftes verlaufend ausgebildet sind und die Rastelemente axial zur Längsachse des Schaftes angeordnet sind.

Zur Anordnung der Rastelemente wird mit einer praktischen Ausführungsform der Erfindung vorgeschlagen, das die Rastelemente in mindestens einer parallel zur mindestens einen Lochscheibe angeordneten Rastscheibe gelagert sind.

Gemäß einer alternativen Ausführungsform der Erfindung wird weiterhin vorgeschlagen, dass in Axialrichtung des Schaftes betrachtet, vor und hinter der mindestens einen Lochscheibe jeweils eine mit Rastelementen versehene Rastscheibe angeordnet ist.

Weiterhin wird mit der Erfindung vorgeschlagen, dass die Rastelemente oder die mindestens eine Lochscheibe zum Lösen der Arretiermechanismus relativ zueinander voneinander fort bewegbar sind. Vorteilhafterweise erfolgt das Aufheben der Arretierung erfindungsgemäß dadurch, dass die mindestens eine Rastscheibe in Axialrichtung des Schaftes von der mindestens einen Lochscheibe fort bewegbar ist.

Um den Schaft in der nicht arretierten Position möglichst einfach in eine neue Position verdrehen zu können, wird gemäß einer vorteilhaften Ausgestaltungsform der Erfindung vorgeschlagen, dass die mindestens eine Rastscheibe zumindest in der die Arretiermechanismus freigebenden Stellung über eine, beispielsweise als Kugelraste ausgebildete Sperrvorrichtung bezüglich der axialen Verschiebbarkeit fixierbar ist.

Zum manuellen Betätigen des Arretiermechanismus wird mit der Erfindung vorgeschlagen, dass die mindestens eine Lochscheibe über eine Stelleinrichtung um die Längsachse des Schaftes drehbar ist.

Um die Handhabung des erfindungsgemäßen Arretiermechanismus besonders einfach auszugestalten, wird weiterhin vorgeschlagen, dass auch die mindestens eine Rastscheibe über die Stelleinrichtung in Axialrichtung des Schaftes verschiebbar ist.

Schließlich wird mit einer alternativen Ausführungsform der Erfindung vorgeschlagen, dass die Ausnehmungen der Lochscheibe in Radialrichtung der Lochscheibe verlaufend ausgebildet sind und die Rastelemente radial sternförmig um die Lochscheibe angeordnet sind. Diese Bauform weist zwar in radialer Richtung einen größeren Durchmesser auf als die axiale Ausrichtung der Rastelemente, jedoch ermöglicht diese Anordnung der Rastelemente eine verkürzte Bauform in axialer Richtung.

Weiterhin wird mit der Erfindung vorgeschlagen, dass der Schaft über einen Kopplungsmechanismus lösbar an der Handhabe festlegbar ist, wodurch die Möglichkeit besteht, den erfindungsgemäß ausgebildeten Schaft je nach Verwendungszweck an unterschiedlichen Handhaben festzulegen.

Weitere Merkmale und Vorteile der Erfindung ergeben sich anhand der zugehörigen Zeichnungen, in denen verschiedene Ausführungsbeispiele eines erfindungsgemäßen medizinischen Instruments nur beispielhaft dargestellt sind, ohne die Erfindung auf diese Ausführungsbeispiele zu beschränken. In den Zeichnungen zeigt:
- Fig. 1: eine Seitenansicht eines erfindungsgemäßen medizinischen Instruments;
- Fig. 2: eine perspektivische Ansicht des vom medizinischen Instrument gelösten Schaftes;
- Fig. 3a: eine vergrößerte Darstellung des Details III gemäß Fig. 2 in einer teilweise transparenten perspektivischen Ansicht in nicht verrasteter Stellung
- Fig. 3b: eine vergrößerte Darstellung des Details III gemäß Fig. 2 in einer teilweise transparenten perspektivischen Ansicht in verrasteter Stellung
- Fig. 4: eine perspektivische Vorderansicht des Arretiermechanismus gemäß Fig. 3;
- Fig. 5: eine Rückansicht der Darstellung gemäß Fig. 4;
- Fig. 6a: eine schematische Seitenansicht einer alternativen Ausführungsform des Arretiermechanismus;
- Fig. 6b: eine schematische Vorderansicht der Rastscheiben des Arretiermechanismus gemäß Fig. 6a und
- Fig. 7: eine schematische Vorderansicht einer weiteren alternativen Ausführungsform des Arretiermechanismus.

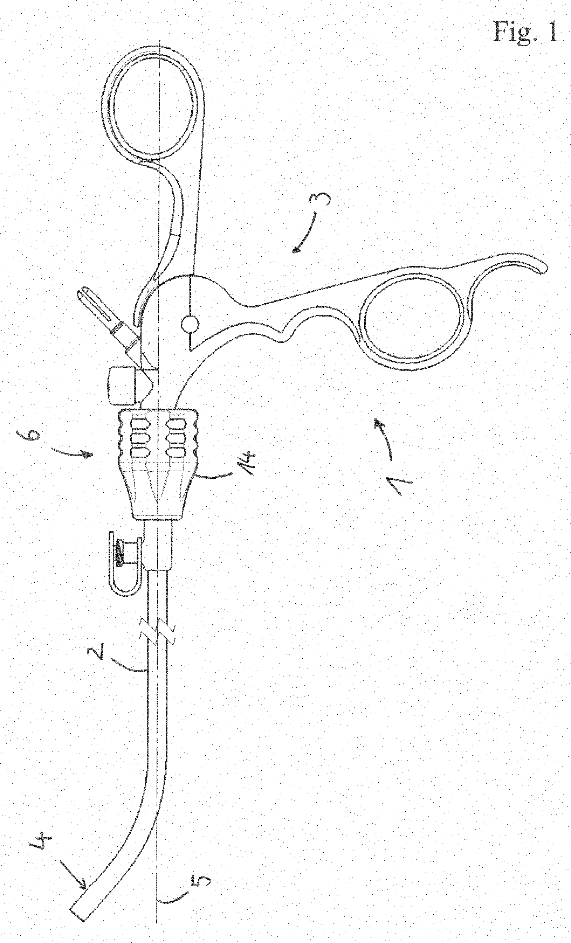

Die Abbildung Fig. 1 zeigt in der Seitenansicht ein medizinisches Instrument 1 mit einem Schaft 2, an dessen proximalem Ende eine Handhabe 3 angeordnet ist. Wie weiterhin aus Fig. 1 ersichtlich, ist der Schaft 2 im Bereich seines distalen Endes 4 gegenüber der Längsachse 5 des Schaftes 2 abgewinkelt ausgebildet. Es sind natürlich auch andere Gestaltungen des Schaftes möglich.

Um das, gemäß dieser Ausführungsform abgewinkelte, distale Ende 4 des Schaftes 2 und somit auch ein in dem hohlen Schaft 2 angeordnetes, nicht dargestelltes, medizinisches Werkzeug positionsgenau positionieren zu können, ist der Schaft 2 um seine Längsachse 5 verdrehbar an der Handhabe 3 gelagert und über einen Arretiermechanismus 6 in verschiedenen Drehstellungen relativ zur Handhabe 3 festlegbar.

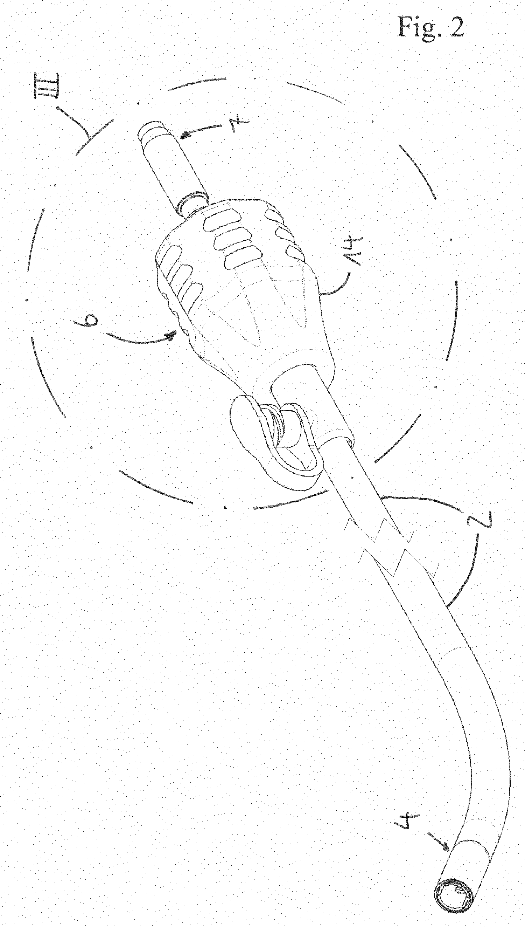

Bei den in den Abbildungen Fig. 2 bis Fig. 7 dargestellten Ausführungsformen ist der Schaft 2 über einen proximalseitig am Schaft 2 angeordneten Kopplungsmechanismus 7 lösbar an der Handhabe festlegbar, wobei der Arretiermechanismus 6 am proximalen Ende des Schaftes 2 angeordnet ist.

Alternativ ist es selbstverständlich auch möglich, den Arretiermechanismus 6 direkt an der Handhabe 3 anzuordnen.

Der Arretiermechanismus 6 ist bei den dargestellten Ausführungsformen als Rastmechanismus ausgebildet, der mindestens eine mit Ausnehmungen 8 versehene Lochscheibe 9 sowie Rastelemente 10 aufweist, wobei die Rastelemente 10 und/oder die mindestens eine Lochscheibe 9 relativ zueinander federbelastet sind und mindestens ein Rastelement 10 zum Fixieren des Schaftes 2 in eine entsprechende Ausnehmung 8 der mindestens einen Lochscheibe 9 eingreift.

Um eine stabile und torsionssteife Fixierung des Schaftes 2 in der den Schaft 2 fixierenden Stellung des Arretiermechanismus 6 zu gewährleisten, greift bei den dargestellten Ausführungsformen immer ein aus zwei Rastelementen 10 bestehendes Rastelementepaar 10 in zwei entsprechende Ausnehmungen 8 der mindestens einen Lochscheibe 9 ein.

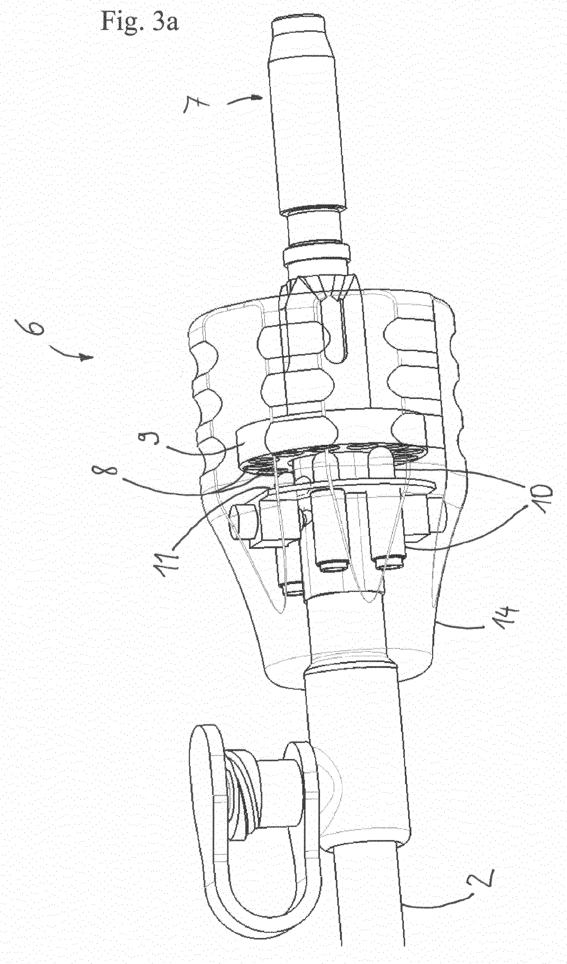

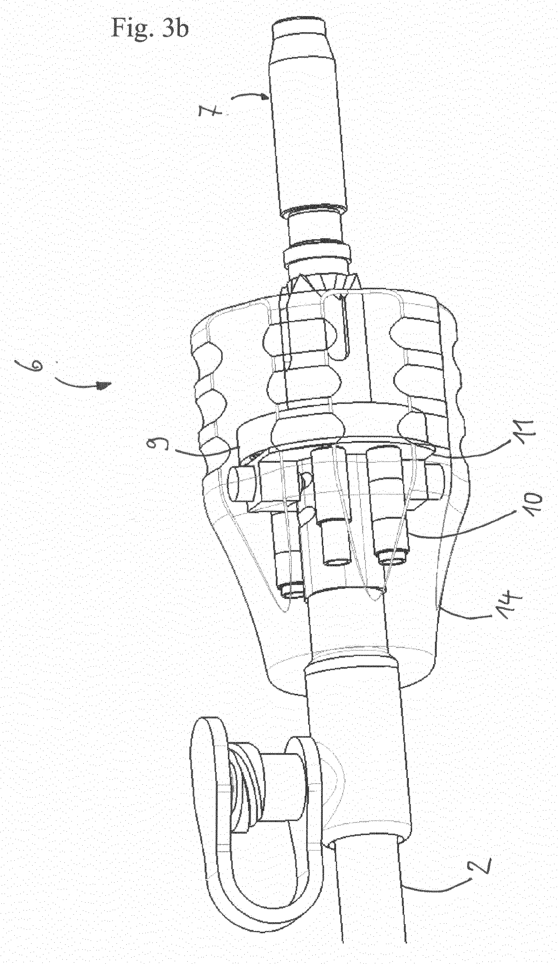

Bei den in den Abbildungen Fig. 3a bis 6b dargestellten Ausführungsformen sind die Ausnehmungen 8 der mindestens einen Lochscheibe 9 in Axialrichtung des Schaftes 2 verlaufend ausgebildet und die Rastelemente 10 axial zur Längsachse 5 des Schaftes 2 angeordnet. Dieses Ausgestaltungsform ermöglicht es, den Durchmesser des medizinischen Instrumente 1 auch im Bereich des Arretiermechanismus 6 möglichst gering ausbilden zu können, was insbesondere bei der Verwendung des medizinischen Instruments 1 für endoskopische Zwecke vorteilhaft ist.

Wie weiterhin aus den Abbildungen Fig. 3a bis Fig. 6b ersichtlich, sind die Rastelemente 10 bei den dargestellten Ausführungsformen in Rastscheiben 11 führend gelagert. Die Rastscheiben 11 gewährleisten eine zielgenaue Ausrichtung der Rastelemente 10 relativ zu den korrespondierenden Ausnehmungen 8 der Lochscheibe 9.

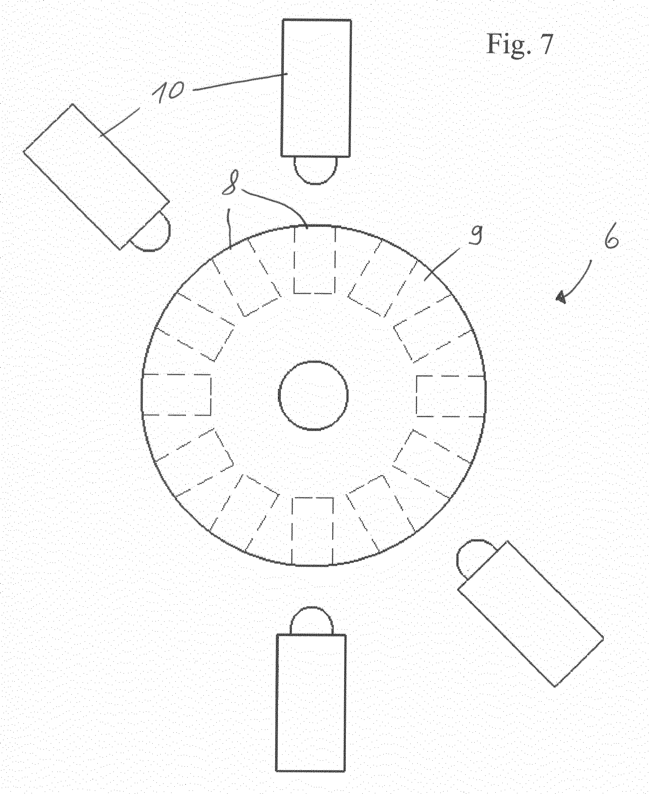

Bei der in Fig. 7 dargestellten alternativen Ausführungsform sind die Ausnehmungen 8 der Lochscheibe 9 in Radialrichtung der Lochscheibe 9 verlaufend ausgebildet und die Rastelemente 10 radial sternförmig um die Lochscheibe 9 angeordnet. Diese Bauform weist zwar in radialer Richtung einen größeren Durchmesser auf als die axiale Ausrichtung der Rastelemente 10 gemäß Fig. 3a bis Fig. 6b, jedoch ermöglicht diese Anordnung der Rastelemente 10 eine verkürzte Bauform des medizinischen Instruments 1 in axialer Richtung.

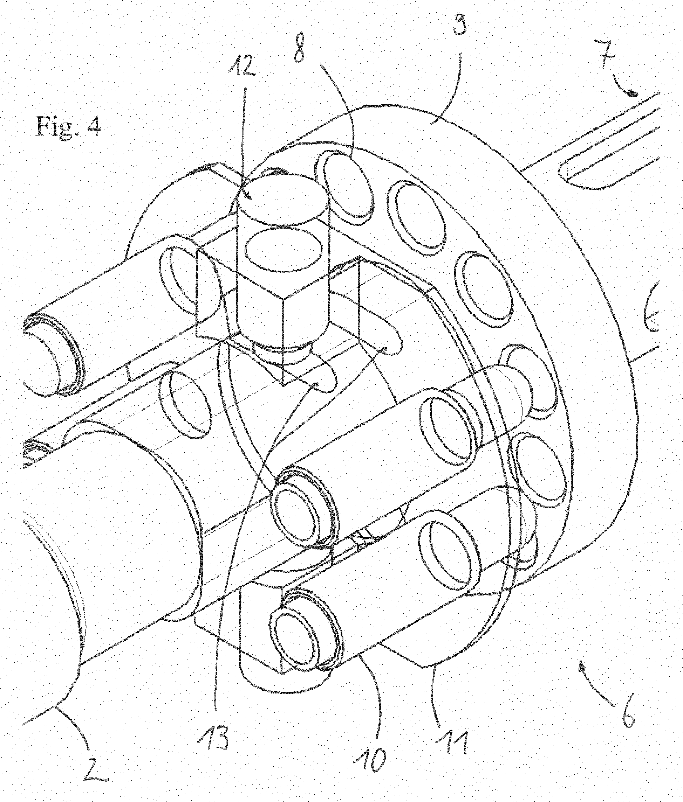

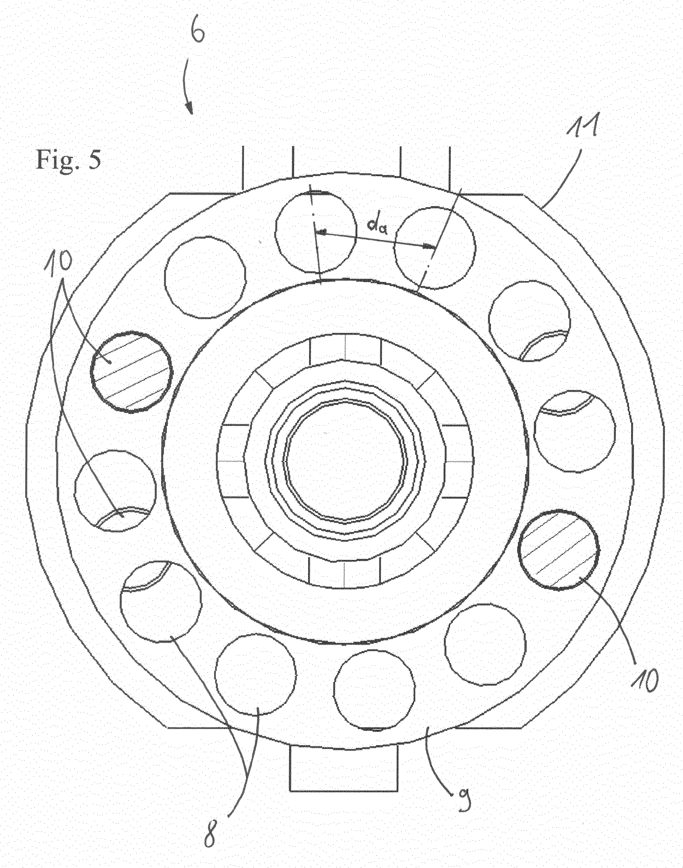

Wie aus Fig. 4 und 5 ersichtlich, greift in der den Schaft 2 fixierenden Stellung jeweils ein Rastelementepaar 10 entsprechende Ausnehmungen 8 der Lochscheibe 9 ein und verhindert so ein weiteres Verdrehen des Schaftes 2 um dessen Längsachse 5. Alle Rastelemente 10 sind dabei über ein nicht dargestelltes Federelement in Richtung auf die Lochscheibe 9 federbelastet, um ein sicheres Verrasten der Rastelemente 10 mit den Ausnehmungen 8 der Lochscheibe 9 zu gewährleisten.

Um möglichst viele positionsgenaue Fixierstufen mit ein und derselben Lochscheibe 9 verwirklichen zu können, sind die Rastelemente 10 so angeordnet, dass nicht alle Rastelemente 10 gleichzeitig Aufnahme in den Ausnehmungen 8 der Lochscheibe 9 finden. Wie insbesondere aus Fig. 5 ersichtlich, nimmt ein aus zwei Rastelementen 10 bestehendes Rastelementepaar 10 eine Zwischenposition ein. Diese Einnahme der Zwischenposition wird durch die Federlagerung der Rastelemente 10 ermöglicht, durch die Rastelemente 10, die nicht mit der Lochscheibe 9 in Eingriff treten, zurückgeschoben werden.

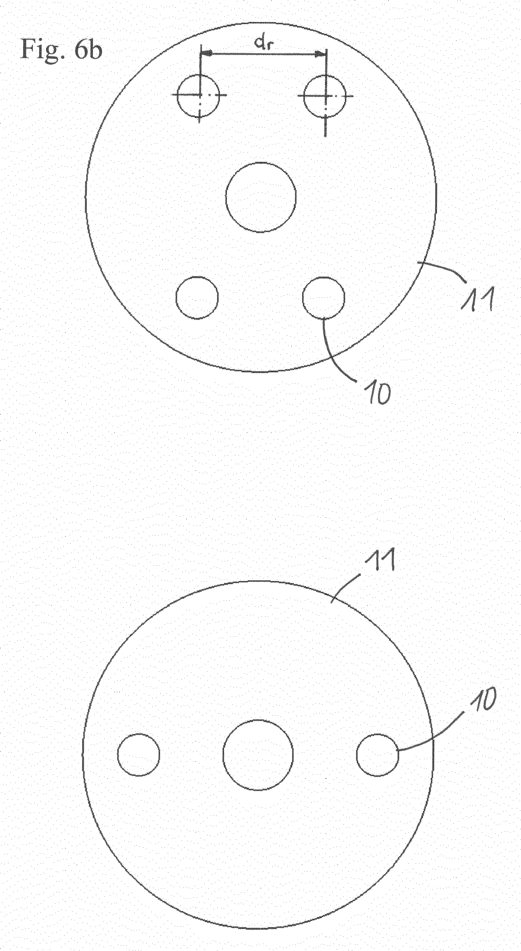

Dadurch, dass sich der Abstand dᵣ zweier nebeneinander angeordneter Rastelemente 10 voneinander, was in Fig. 6b abgebildet ist, vom Abstand dₐ zweier nebeneinander angeordneter Ausnehmungen 8 der Lochscheibe 9, was in Fig. 5 abgebildet ist, unterscheidet, lassen sich beispielsweise bei zwei Rastelementepaaren, also vier einzelnen Rastelementen, und zwölf Ausnehmungen 8 in der Lochscheibe 9 insgesamt vierundzwanzig Fixierungsstufen verwirklichen.

Zum Lösen des Arretiermechanismus 6 sind die Rastscheibe 11 oder die Lochscheibe 9 relativ zueinander voneinander fort bewegbar auf dem Schaft 2 angeordnet. Vorteilhafterweise erfolgt das Aufheben der Arretierung wie in Fig. 4 dargestellt dadurch, dass die Rastscheibe 11 in Axialrichtung des Schaftes 2 von der Lochscheibe 9 fort bewegbar ist.

Damit der Schaft 2 in der nicht arretierten Position möglichst einfach in eine neue Position verdreht werden kann, ist die Rastscheibe 11, wie aus Fig. 4 ersichtlich, zumindest in der die Arretiermechanismus 6 freigebenden Stellung über eine, beispielsweise als Kugelraste ausgebildete Sperrvorrichtung 12 bezüglich der axialen Verschiebbarkeit fixierbar ist. Vorteilhafterweise ist die Sperreinrichtung 12 aber so ausgebildet, dass die Rastscheibe 11 sowohl in der den Arretiermechanismus 6 freigebenden als auch in der arretierenden Position axial fixierbar ist. Hierzu weist der Schaft 2 Kerben 13 auf, in die die Sperreinrichtung 12 in der jeweiligen Position eingreift.

Zum manuellen Betätigen des Arretiermechanismus 6 gemäß Fig. 4 und 5 ist die Lochscheibe 9 über eine, vorzugsweise als Drehrad ausgebildete Stelleinrichtung 14 um die Längsachse 5 des Schaftes 2 drehbar.

Um die Handhabung des Arretiermechanismus 6 besonders einfach auszugestalten, ist die Stelleinrichtung 14 vorzugsweise so ausgebildet, dass auch das axiale Verschieben der Rastscheibe 11 über die Stelleinrichtung 14 erfolgen kann.

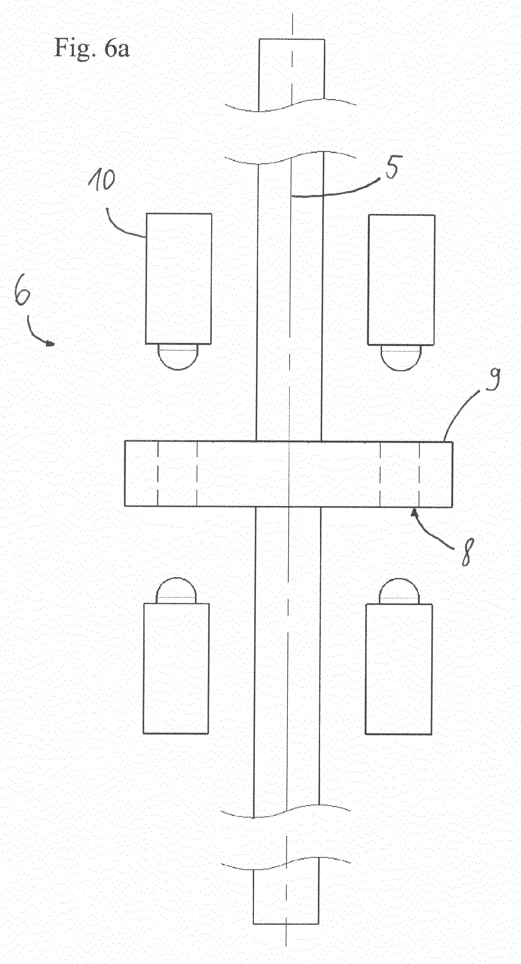

Die in den Abbildungen Fig. 6a und 6b dargestellte alternative Ausführungsform zur Ausbildung des Arretiermechanimus 6 unterscheidet sich von der Anordnung gemäß Fig. 4 und 5 dadurch, dass bei dieser zweiten Ausführungsform des Arretiermechanismus 6 die Rastelemente 10 in Rastscheiben 11 gelagert sind, die in Axialrichtung des Schaftes 2 betrachtet, vor und hinter der Lochscheibe 9 angeordnet sind. Um die Anzahl der möglichen Fixierungsstufen zu erhöhen, weisen die beiden Rastscheiben 11, wie aus Fig. 6b ersichtlich, verschiedene Muster zur Anordnung der Rastelemente 10 auf.

Alternativ zu der Federbelastung der Rastelemente 10 kann die Verrastung zwischen den Rastelementen 10 und den Ausnehmungen 8 der Lochscheibe 9 bei der in Fig. 6a und 6b dargestellten Ausführungsform auch dadurch erreicht werden, dass die Lochscheibe 9 in Axialrichtung verschiebbar auf dem Schaft 2 gelagert ist und so in Richtung der beiden Rastscheiben 11 verschoben werden kann.

Ein wie voranstehend beschrieben aufgebautes medizinisches Instrument 1 zeichnet sich dadurch aus, dass der Schaft 2 bei einfacher Handhabung sicherer und positionsgenau in eine Vielzahl fester Positionen um seine Längsachse 5 verdreht werden kann.

### Bezugszeichenliste

- 1: medizinisches Instrument
- 2: Schaft
- 3: Handhabe
- 4: distales Ende (Schaft)
- 5: Längsachse
- 6: Arretiermechanismus
- 7: Kopplungsmechanismus
- 8: Ausnehmung
- 9: Lochscheibe
- 10: Rastelement / Rastelementepaar
- 11: Rastscheibe
- 12: Sperreinrichtung
- 13: Kerbe
- 14: Stelleinrichtung

- dᵣ: Abstand (Rastelemente)
- dₐ: Abstand (Ausnehmungen)

## Patentansprüche

1. Medizinisches Instrument mit einem Schaft (2), an dessen proximalem Ende eine Handhabe (3) angeordnet ist, wobei der Schaft (2) um seine Längsachse (5) verdrehbar an der Handhabe (2) gelagert ist und über einen Arretiermechanismus (6) in verschiedenen Drehstellungen relativ zur Handhabe (3) festlegbar ist, wobei der Arretiermechanismus (6) als Rastmechanismus ausgebildet ist, der mindestens eine mit Ausnehmungen (8) versehene Lochscheibe (9) sowie Rastelemente (10) aufweist, wobei die Rastelemente (10) und/oder die mindestens eine Lochscheibe (9) relativ zueinander federbelastet sind und mindestens ein Rastelement (10) zum Fixieren des Schaftes (2) in eine entsprechende Ausnehmung (8) der mindestens einen Lochscheibe (9) eingreift,
wobei die Rastelemente (10) in Richtung auf die mindestens eine Lochscheibe (9) federbelastet sind,
**dadurch gekennzeichnet, dass**
der Abstand (dᵣ) zweier nebeneinander angeordneter Rastelemente (10) voneinander sich vom Abstand (dₐ) zweier nebeneinander angeordneter Ausnehmungen (8) der mindestens einen Lochscheibe (9) unterscheidet.

2. Medizinisches Instrument nach Anspruch 1, **dadurch gekennzeichnet, dass** in der den Schaft (2) fixierenden Stellung des Arretiermechanismus (6) immer ein aus zwei Rastelementen (10) bestehendes Rastelementepaar (10) in zwei entsprechende Ausnehmungen (8) der mindestens einen Lochscheibe (9) eingreift.

3. Medizinisches Instrument nach Anspruch 2, **dadurch gekennzeichnet, dass** der Arretiermechanismus (6) mehrere nebeneinander angeordnete Rastelementepaare (10) aufweist.

4. Medizinisches Instrument nach Anspruch 3, **dadurch gekennzeichnet, dass** der Abstand (dᵣ) zweier nebeneinander angeordneter Rastelementepaare (10) voneinander sich vom Abstand (dₐ) zweier nebeneinander angeordneter Ausnehmungen (8) der mindestens einen Lochscheibe (9) unterscheidet.

5. Medizinisches Instrument nach einem der Ansprüche 1 bis 4, **dadurch gekennzeichnet, dass** die Ausnehmungen (8) der mindestens einen Lochscheibe (9) in Axialrichtung des Schaftes (2) verlaufend ausgebildet sind und die Rastelemente (10) axial zur Längsachse (5) des Schaftes (2) angeordnet sind.

6. Medizinisches Instrument nach Anspruch 5, **dadurch gekennzeichnet, dass** die Rastelemente (10) in mindestens einer parallel zur mindestens einen Lochscheibe (9) angeordneten Rastscheibe (11) gelagert sind.

7. Medizinisches Instrument nach Anspruch 6, **dadurch gekennzeichnet, dass** in Axialrichtung des Schaftes (2) betrachtet, vor und hinter der mindestens einen Lochscheibe (9) jeweils eine mit Rastelementen (10) versehene Rastscheibe (11) angeordnet ist.

8. Medizinisches Instrument nach einem der Ansprüche 1 bis 7, **dadurch gekennzeichnet, dass** die Rastelemente (10) oder die mindestens eine Lochscheibe (9) zum Lösen des Arretiermechanismus (6) relativ zueinander voneinander fort bewegbar sind.

9. Medizinisches Instrument nach den Ansprüchen 6 und 8, **dadurch gekennzeichnet, dass** die mindestens eine Rastscheibe (11) in Axialrichtung des Schaftes (2) von der mindestens einen Lochscheibe (9) fort bewegbar ist.

10. Medizinisches Instrument nach Anspruch 9, **dadurch gekennzeichnet, dass** die mindestens eine Rastscheibe (11) zumindest in der die Arretiermechanismus (6) freigebenden Stellung über eine Sperrvorrichtung (12) bezüglich der axialen Verschiebbarkeit fixierbar ist.

11. Medizinisches Instrument nach Anspruch 10, **dadurch gekennzeichnet, dass** die Sperrvorrichtung (12) als Kugelraste ausgebildet ist.

12. Medizinisches Instrument nach einem der Ansprüche 1 bis 11, **dadurch gekennzeichnet, dass** die mindestens eine Lochscheibe (9) über eine Stelleinrichtung (14) um die Längsachse (5) des Schaftes (2) drehbar ist.

13. Medizinisches Instrument nach Anspruch 12, **dadurch gekennzeichnet, dass** die mindestens eine Rastscheibe (11) über die Stelleinrichtung (14) in Axialrichtung des Schaftes (2) verschiebbar ist.

14. Medizinisches Instrument nach einem der Ansprüche 1 bis 3, **dadurch gekennzeichnet, dass** die Ausnehmungen (8) der Lochscheibe (9) in Radialrichtung der Lochscheibe (9) verlaufend ausgebildet sind und die Rastelemente (10) radial sternförmig um die Lochscheibe (9) angeordnet sind.

15. Medizinisches Instrument nach einem der Ansprüche 1 bis 3, **dadurch gekennzeichnet, dass** der Schaft (2) über einen Kopplungsmechanismus (7) lösbar an der Handhabe (3) festlegbar ist.

## Claims

1. Medical instrument with a shank (2), at the proximal end of which a handle (3) is arranged, wherein the shank (2) is mounted so as to be rotatable about its longitudinal axis (5) on the handle (2) and can be secured in various positions of rotation relative to the handle (3) via a locking mechanism (6), wherein the locking mechanism (6) is designed as a latching mechanism which has at least one perforated disk (9), provided with recesses (8), and also latching elements (10), wherein the latching elements (10) and/or the at least one perforated disk (9) are spring-loaded relative to each other, and at least one latching element (10) engages in a corresponding recess (8) of the at least one perforated disk (9) in order to fix the shank (2), wherein the latching elements (10) are spring-loaded in the direction of the at least one perforated disk (9),
**characterized in that**
the distance (dᵣ) between two latching elements (10) arranged next to each other differs from the distance (dₐ) between two recesses (8) arranged next to each other on the at least one perforated disk (9).

2. Medical instrument according to Claim 1, **characterized in that**, in the position of the locking mechanism (6) in which it fixes the shank (2), a latching element pair (10) consisting of two latching elements (10) always engages in two corresponding recesses (8) of the at least one perforated disk (9).

3. Medical instrument according to Claim 2, **characterized in that** the locking mechanism (6) has a plurality of latching element pairs (10) arranged next to each other.

4. Medical instrument according to Claim 3, **characterized in that** the distance (dᵣ) between two latching element pairs (10) arranged next to each other differs from the distance (dₐ) between two recesses (8) arranged next to each other on the at least one perforated disk (9).

5. Medical instrument according to one of Claims 1 to 4, **characterized in that** the recesses (8) of the at least one perforated disk (9) are designed extending in the axial direction of the shank (2), and the latching elements (10) are arranged axially with respect to the longitudinal axis (5) of the shank (2).

6. Medical instrument according to Claim 5, **characterized in that** the latching elements (10) are mounted in at least one latching disk (11) arranged parallel to the at least one perforated disk (9).

7. Medical instrument according to Claim 6, **characterized in that** a latching disk (11) provided with latching elements (10) is in each case arranged upstream and downstream of the at least one perforated disk (9) as seen in the axial direction of the shank (2).

8. Medical instrument according to one of Claims 1 to 7, **characterized in that** the latching elements (10) or the at least one perforated disk (9) are movable relative to each other in a direction away from each other in order to release the locking mechanism (6).

9. Medical instrument according to Claims 6 and 8, **characterized in that** the at least one latching disk (11) is movable away from the at least one perforated disk (9) in the axial direction of the shank (2).

10. Medical instrument according to Claim 9, **characterized in that** the at least one latching disk (11), at least in the position releasing the locking mechanism (6), can be fixed with respect to the axial mobility via a catch device (12).

11. Medical instrument according to Claim 10, **characterized in that** the catch device (12) is designed as a ball catch.

12. Medical instrument according to one of Claims 1 to 11, **characterized in that** the at least one perforated disk (9) is rotatable about the longitudinal axis (5) of the shank (2) via an adjustment device (14).

13. Medical instrument according to Claim 12, **characterized in that** the at least one latching disk (11) is movable in the axial direction of the shank (2) via the adjustment device (14).

14. Medical instrument according to one of Claims 1 to 3, **characterized in that** the recesses (8) of the perforated disk (9) are designed extending in the radial direction of the perforated disk (9), and the latching elements (10) are arranged radially in a star shape around the perforated disk (9).

15. Medical instrument according to one of Claims 1 to 3, **characterized in that** the shank (2) can be secured releasably on the handle (3) via a coupling mechanism (7).

## Revendications

1. Instrument médical comprenant une tige (2) à l'extrémité proximale de laquelle est disposée une manette (3), la tige (2) étant supportée de manière à pouvoir tourner autour de son axe longitudinal (5) sur la manette (2) et pouvant être fixée par le biais d'un mécanisme d'arrêt (6) dans différentes positions de rotation par rapport à la manette (3), le mécanisme d'arrêt (6) étant réalisé sous forme de mécanisme d'encliquetage qui présente au moins un disque perforé (9) pourvu d'évidements (8) ainsi que des éléments d'encliquetage (10), les éléments d'encliquetage (10) et/ou l'au moins un disque perforé (9) étant sollicités par ressort les uns par rapport aux autres et au moins un élément d'encliquetage (10), pour fixer la tige (2), s'engageant dans un évidement correspondant (8) de l'au moins un disque perforé (9),
les éléments d'encliquetage (10) étant sollicités par ressort dans la direction de l'au moins un disque perforé (9),
**caractérisé en ce que**
la distance (dᵣ) entre deux éléments d'encliquetage (10) adjacents est différente de la distance (dₐ) entre deux évidements adjacents (8) de l'au moins un disque perforé (9).

2. Instrument médical selon la revendication 1, **caractérisé en ce que** dans la position du mécanisme d'arrêt (6) fixant la tige (2) une paire d'éléments d'encliquetage (10) constituée de deux éléments d'encliquetage (10) s'engage toujours dans deux évidements correspondants (8) de l'au moins un disque perforé (9).

3. Instrument médical selon la revendication 2, **caractérisé en ce que** le mécanisme d'arrêt (6) présente plusieurs paires d'éléments d'encliquetage (10) disposées les unes à côté des autres.

4. Instrument médical selon la revendication 3, **caractérisé en ce que** la distance (dᵣ) entre deux paires d'éléments d'encliquetage adjacentes (10) est différente de la distance (dₐ) entre deux évidements adjacents (8) de l'au moins un disque perforé (9).

5. Instrument médical selon l'une quelconque des revendications 1 à 4, **caractérisé en ce que** les évidements (8) de l'au moins un disque perforé (9) sont réalisés de manière à s'étendre dans la direction axiale de la tige (2) et les éléments d'encliquetage (10) sont disposés axialement par rapport à l'axe longitudinal (5) de la tige (2).

6. Instrument médical selon la revendication 5, **caractérisé en ce que** les éléments d'encliquetage (10) sont supportés dans au moins un disque d'encliquetage (11) disposé parallèlement à au moins un disque perforé (9).

7. Instrument médical selon la revendication 6, **caractérisé en ce que**, vu dans la direction axiale de la tige (2), avant et derrière l'au moins un disque perforé (9), est à chaque fois disposé un disque d'encliquetage (11) pourvu d'éléments d'encliquetage (10).

8. Instrument médical selon l'une quelconque des revendications 1 à 7, **caractérisé en ce que** les éléments d'encliquetage (10) ou l'au moins un disque perforé (9) peuvent être éloignés l'un par rapport à l'autre pour desserrer le mécanisme d'arrêt (6).

9. Instrument médical selon les revendications 6 et 8, **caractérisé en ce que** l'au moins un disque d'encliquetage (11) peut être déplacé dans la direction axiale de la tige (2) à l'écart de l'au moins un disque perforé (9).

10. Instrument médical selon la revendication 9, **caractérisé en ce que** l'au moins un disque d'encliquetage (11) peut être fixé par rapport à sa mobilité axiale au moins dans la position libérant le mécanisme d'arrêt (6) par le biais d'un dispositif de blocage (12).

11. Instrument médical selon la revendication 10, **caractérisé en ce que** le dispositif de blocage (12) est réalisé sous forme de loqueteau à bille.

12. Instrument médical selon l'une quelconque des revendications 1 à 11, **caractérisé en ce que** l'au moins un disque perforé (9) peut tourner autour de l'axe longitudinal (5) de la tige (2) par le biais d'un dispositif de réglage (14).

13. Instrument médical selon la revendication 12, **caractérisé en ce que** l'au moins un disque d'encliquetage (11) peut être déplacé dans la direction axiale de la tige (2) par le biais du dispositif de réglage (14).

14. Instrument médical selon l'une quelconque des revendications 1 à 3, **caractérisé en ce que** les évidements (8) du disque perforé (9) sont réalisés de manière à s'étendre dans la direction radiale du disque perforé (9) et les éléments d'encliquetage (10) sont disposés radialement en forme d'étoile autour du disque perforé (9).

15. Instrument médical selon l'une quelconque des revendications 1 à 3, **caractérisé en ce que** la tige (2) peut être fixée à la manette (3) de manière desserrable par le biais d'un mécanisme d'accouplement (7).
